# EUROPEAN PATENT APPLICATION

(11) **EP 4 434 578 A1**
(43) Date of publication of application: **25.09.2024**
(21) Application number: 22895906.0
(22) Date of filing: 02.11.2022
(51) Int. Cl.: A61N 7/02, B06B 1/06, A61N 7/00

(54) **METHOD FOR MANUFACTURING HIGH-INTENSITY FOCUSED ULTRASOUND PROBE FOR SKIN THERAPY**

(30) Priority: 17.11.2021 KR 20210158796
(71) Applicant: VIOL Co. Ltd., Seongnam-si, Gyeonggi-do 13510 (KR); Daegu Gyeongbuk Institute Of Science and Technology, Daegu 42988 (KR); Dong IL Technology Ltd., Hwaseong-si, Gyeonggi-do 18255 (KR)
(72) Inventor: CHANG, Jin Ho, Seoul 07997 (KR); KIM, Jin Woo, Seoul 04096 (KR); KIM, Ju Hwan, Gwangmyeong-si Gyeonggi-do 14318 (KR); NA, Jong Ju, Seoul 05647 (KR); LEE, Duk Kyu, Seoul 04947 (KR); KIM, Joung Pil, Hwaseong-si Gyeonggi-do 18322 (KR)
(74) Representative: Murgitroyd & Company
(86) International application number: PCT/KR2022/016969
(87) International publication number: WO 2023/090699

(57) **Abstract**

Disclosed is a method of manufacturing a high-intensity focused ultrasound probe including manufacturing a transducer module configured such that a plurality of transducer elements having a concave shape in a first axis direction, which is a focusing direction of ultrasonic waves generated from the transducer module by receiving an electrical signal, is arranged in a second axis direction perpendicular to the first axis direction with kerfs interposed between the transducer elements, and disposing a circuit configured to input the electrical signal to the transducer module, each of the plurality of transducer elements being formed in a geometrical structure configured to cause the ultrasonic waves generated by receiving the electrical signal to be focused on a predetermined point and having a shape configured such that a length thereof in a third axis direction perpendicular to the first axis direction and the second axis direction is longer than a length thereof in the second axis direction

## Description

### [Technical Field]

The present disclosure relates to a method of manufacturing a high-intensity focused ultrasound probe (HIFU), and more particularly, to a method of manufacturing a high-intensity focused ultrasound probe capable of focusing high-intensity ultrasonic waves for skin treatment based on the geometrical structure of transducer elements.

### [Background Art]

Recently, use of high-intensity focused ultrasound (HIFU) for skin treatment, such as skin lifting and skin tightening, has received much attention.

High-intensity focused ultrasound is a technique that treats lesions by focusing high-intensity acoustic energy on a local area of the body to increase the temperature of the local area at the focusing point and generate thermal variation. When this technique is used on the skin, effects of removing wrinkles and promoting restoration of skin elasticity through regeneration of tissues denatured by a wound created due to thermal variation in a local area, on which fine ultrasonic waves are focused, are exhibited.

A conventional high-intensity focused ultrasound apparatus is a transducer which generates ultrasonic waves, and uses a method of transferring strong ultrasonic energy to a treatment area using a circular single-element ultrasound transducer. In order to transfer ultrasonic energy for skin treatment to a wide area, a plurality of focusing points must be formed, and therefore, the conventional single-element ultrasound transducer uses a method of mechanically moving the single-element ultrasound transducer to focus ultrasonic waves on various body parts.

Therefore, the conventional high-intensity focused ultrasound apparatus adopts the circular single-element ultrasound transducer, and thus, if it is applied to different treatment depths (focusing points), handpieces designed with different ultrasound frequencies and geometrical structures of transducers need to be replaced every time. In order to solve this problem, prior art document 1 discloses that a lever, a motor, or a scanner is mounted at the tip of a handpiece and allows a user to arbitrarily select the depth of a target site manually, semi-automatically, or fully automatically to focus ultrasonic waves on the target site. However, the prior art still requires mechanical movement because a transducer must still be moved using the motor. Further, the mechanical movement results in a long treatment time and requires mechanical change in the position of the ultrasound transducer in the handpiece to change a treatment depth, thereby limiting improvement in treatment effects.

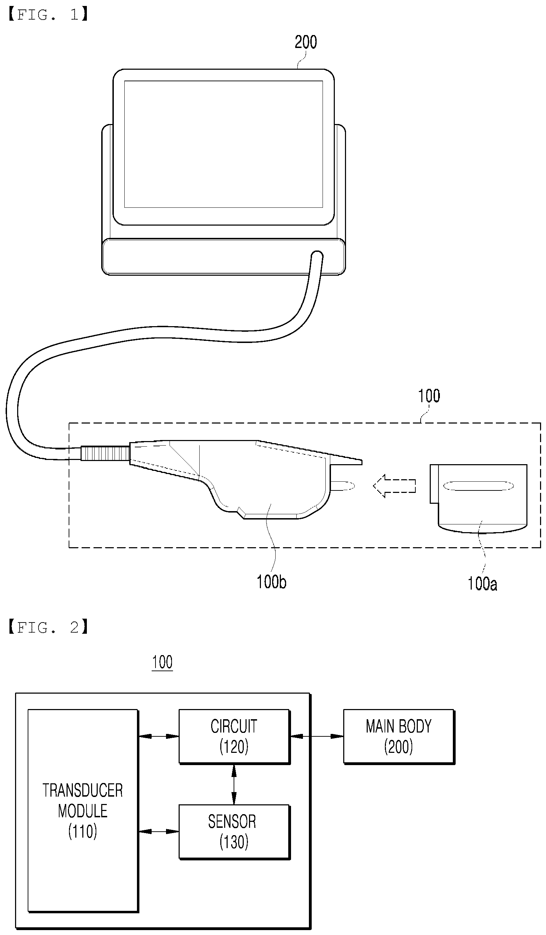

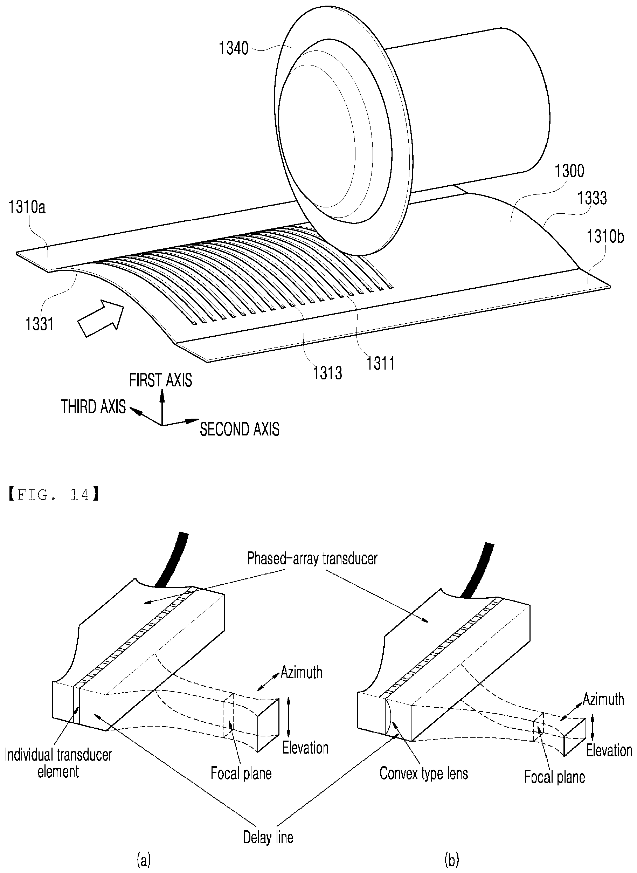

In contrast, a diagnostic ultrasound apparatus which generates small ultrasonic energy, uses array-type ultrasound transducers, as shown in FIG. 1. That is, ultrasonic waves are focused on a desired position by electrically controlling the transmission time of a plurality of ultrasound transducers arranged in a lateral direction (an x-axis direction) is electrically controlled, and reflected ultrasonic waves are received and imaged. Here, in order to improve diagnostic resolution, a focusing technique may be used in the elevation direction (y-axis direction) to transfer high ultrasonic energy to a focal point, and as a focusing method, a natural focusing method, as shown in FIG. 14(a), or an acoustic lens focusing method, as shown in FIG. 14(b), is used.

Even in the case of the conventional diagnostic ultrasound apparatus, geometrical focusing may not be used in the array-type ultrasound transducers due to a difficulty in the manufacturing process thereof, and the above-described high-intensity focused ultrasound apparatus, which is an ultrasound apparatus for treatment, uses only a single ultrasound transducer element.

Due to these problems, it is difficult to implement a high-intensity focused ultrasound apparatus using array-type ultrasound transducer elements. Natural focusing is unsuitable because energy is dispersed, and therefore, acoustic lens focusing or geometrical focusing is suitable to increase the temperature of a lesion. However, in the case of lens focusing, it is difficult to transfer high-intensity energy to a treatment area due to ultrasonic wave attenuation caused by a material used as the lens, and it is difficult to form a geometrical structure of array-type ultrasound transducers to enable geometrical focusing (in order to control the electrical transmission time of the array-type ultrasound transducers, at least dozens of ultrasound transducers having at least a focusable geometrical structure must be provided).

### [Disclosure]

### [Technical Problem]

Therefore, the present disclosure has been made in view of the above problems, and it is an object of the present disclosure to provide a high-intensity focused ultrasound probe in which a plurality of transducers capable of geometrical focusing based on a geometrical structure is arranged in an array type, and a method of manufacturing the same.

It is another object of the present disclosure to provide a high-intensity focused ultrasound probe in which a plurality of transducers capable of geometrical focusing, which may be easily manufactured at a low cost, is arranged in an array type, and a method of manufacturing the same.

It is yet another object of the present disclosure to provide a high-intensity focused ultrasound probe in which a plurality of transducers capable of geometrical focusing, which may transmit high energy to a treatment site while reducing a grating lobe level, is arranged in an array type, and a method of manufacturing the same.

It is still another object of the present disclosure to provide a high-intensity focused ultrasound probe in which the transmission time of each of a plurality of ultrasound transducers arranged in an array type may be electrically controlled to shorten a treatment time without mechanical movement, and a method of manufacturing the same.

### [Technical Solution]

One embodiment of the present disclosure provides a high-intensity focused ultrasound probe and a method of manufacturing the same.

The high-intensity focused ultrasound probe according to one embodiment of the present disclosure may include a transducer module configured to receive an electrical signal and generate ultrasonic waves, and a circuit configured to input the electrical signal to the transducer module, the transducer module may be provided in a form in which a plurality of transducer elements having a concave shape in a first axis direction, which is a focusing direction of the generated ultrasonic waves, is arranged in a second axis direction perpendicular to the first axis direction with kerfs interposed between the transducer elements, and each of the plurality of transducer elements may be formed in a geometrical structure configured to cause the ultrasonic waves generated by receiving the electrical signal to be focused on a predetermined point, and may have a shape configured such that a length thereof in a third axis direction perpendicular to the first axis direction and the second axis direction is longer than a length thereof in the second axis direction.

The method of manufacturing the high-intensity focused ultrasound probe according to one embodiment of the present disclosure may include manufacturing a transducer module configured such that a plurality of transducer elements having a concave shape in a first axis direction, which is a focusing direction of ultrasonic waves generated from the transducer module by receiving an electrical signal, is arranged in a second axis direction perpendicular to the first axis direction with kerfs interposed between the transducer elements, and disposing a circuit configured to input the electrical signal to the transducer module, and each of the plurality of transducer elements may be formed in a geometrical structure configured to cause the ultrasonic waves generated by receiving the electrical signal to be focused on a predetermined point, and may have a shape configured such that a length thereof in a third axis direction perpendicular to the first axis direction and the second axis direction is longer than a length thereof in the second axis direction.

The high-intensity focused ultrasound probe according to one embodiment of the present disclosure may include a transducer module configured to receive an electrical signal and generate ultrasonic waves, and a circuit configured to input the electrical signal to the transducer module, and the transducer module may include a curved transducer part having a concave shape in a first axis direction, which is a focusing direction of the generated ultrasonic waves, a plurality of kerfs, arranged in a second axis direction perpendicular to the first axis direction in the curved transducer part, provided to extend lengthwise in a third axis direction perpendicular to the first axis direction and the second axis direction, and configured to separate at least portions of the curved transducer part so as to form transducer elements at a plurality of regions, and a bent part configured to be bent in a direction opposite to the focusing direction of the ultrasonic waves and formed at least one end of both ends of the transducer part in the third axis direction.

The method of manufacturing the high-intensity focused ultrasound probe according to one embodiment of the present disclosure may include forming a transducer module configured to receive an electrical signal and generate ultrasonic waves, and disposing a circuit configured to input the electrical signal to the transducer module, and forming the transducer module may include forming a curved transducer part having a concave shape in a first axis direction, which is a focusing direction of the generated ultrasonic waves, forming a plurality of kerfs, arranged in a second axis direction perpendicular to the first axis direction in the curved transducer part, provided to extend lengthwise in a third axis direction perpendicular to the first axis direction and the second axis direction, and configured to separate at least portions of the curved transducer part so as to form transducer elements at a plurality of regions, and forming a bent part, configured to be bent in a direction opposite to the focusing direction of the ultrasonic waves, at least one end of both ends of the transducer part in the third axis direction.

The method of manufacturing the high-intensity focused ultrasound probe according to one embodiment of the present disclosure may include providing a plurality of transducer elements having a predetermined first thickness in a first axis direction and a predetermined curvature to be concave in the first axis direction, and configured to extend lengthwise in a third axis direction perpendicular to the first axis direction, providing a plurality of plate-type separation members having a predetermined second thickness, and alternately stacking the plurality of transducer elements and the plurality of separation members in a second direction perpendicular to the first axis direction and the third axis direction.

The method of manufacturing the high-intensity focused ultrasound probe according to one embodiment of the present disclosure may include providing a plurality of plate-type transducer elements having a predetermined first thickness in a second axis direction, providing a plurality of plate-type separation members having a predetermined second thickness, forming an assembly by alternately stacking the plurality of plate-type transducer elements and the plurality of plate-type separation members in the second axis direction, and grinding one surface or both surfaces of the assembly perpendicular a first axis direction so that the plurality of plate-type transducer elements has a predetermined curvature to be concave in the first axis direction perpendicular to the second axis direction and extends in a third axis direction perpendicular to the first axis direction and the second axis direction.

The method of manufacturing the high-intensity focused ultrasound probe according to one embodiment of the present disclosure may include providing a transducer element unit including a curved transducer part configured such that at least a portion thereof has a concave shape in a first axis direction, which is a focusing direction of ultrasonic waves, and bent parts configured to bent in a direction opposite to the focusing direction of the ultrasonic waves from both ends of the transducer part in a third axis direction perpendicular to the first axis direction, and forming a plurality of kerfs, configured to extend lengthwise in the third axis direction and arranged in a second axis direction perpendicular to the first axis direction and the third axis direction, in the curved transducer part by cutting at least portions of the curved transducer part.

### [Advantageous effects]

A high-intensity focused ultrasound probe and a method of manufacturing the same according to embodiments of the present disclosure may allow ultrasonic waves to be geometrically focused based on the geometrical structure of a plurality of high-intensity focused ultrasound transducers, and may electrically control a transmission time, thereby being capable of easily providing an array-type high-intensity focused ultrasound probe at a low cost and shortening a treatment time while improving a treatment effect.

### [Description of Drawings]

FIG. 1 is a view illustrating a high-intensity focused ultrasound probe according to one embodiment of the present disclosure.
FIG. 2 is a block diagram briefly illustrating the configuration of the high-intensity focused ultrasound probe according to one embodiment of the present disclosure.
FIG. 3 shows a perspective view and a plan view illustrating a transducer module of the array-type high-intensity focused ultrasound probe according to one embodiment of the present disclosure.
FIG. 4 shows a perspective view and a plan view illustrating the transducer module of the array-type high-intensity focused ultrasound probe according to one embodiment of the present disclosure.
FIG. 5 shows results of tests on kerf widths of the array-type high-intensity focused ultrasound probe according to one embodiment of the present disclosure.
FIG. 6 shows results of tests on element widths of the array-type high-intensity focused ultrasound probe according to one embodiment of the present disclosure.
FIG. 7 is a perspective view showing a transducer module of an array-type high-intensity focused ultrasound probe according to another embodiment of the present disclosure.
FIG. 8 is a flowchart for explaining a method of manufacturing an array-type high-intensity focused ultrasound probe according to one embodiment of the present disclosure.
FIG. 9 is a view illustrating the method of manufacturing the array-type high-intensity focused ultrasound probe according to one embodiment of the present disclosure.
FIG. 10 is a flowchart for explaining a method of manufacturing an array-type high-intensity focused ultrasound probe according to another embodiment of the present disclosure.
FIG. 11 is a view illustrating the method of manufacturing the array-type high-intensity focused ultrasound probe according to another embodiment of the present disclosure.
FIG. 12 is a flowchart for explaining a method of manufacturing an array-type high-intensity focused ultrasound probe according to yet another embodiment of the present disclosure.
FIG. 13 is a view illustrating the method of manufacturing the array-type high-intensity focused ultrasound probe according to yet another embodiment of the present disclosure.
FIG. 14 is a view illustrating focusing methods of probes used in conventional diagnostic ultrasound imaging devices.

### [Mode for Invention]

Hereinafter, reference will be made in detail to exemplary embodiments of the present disclosure, examples of which are illustrated in the accompanying drawings and described below, and wherever possible, the same or similar elements will be denoted by the same reference numerals even though they are depicted in different drawings and a redundant description thereof will thus be omitted. In the following description of the embodiments, suffixes, such as "module", and "part", are provided or used interchangeably merely in consideration of ease in statement of the specification, and do not have meanings or functions distinguished from one another. In the following description of the embodiments of the present disclosure, a detailed description of known functions and configurations incorporated herein will be omitted when it may make the subject matter of the present disclosure rather unclear. Further, the accompanying drawings will be exemplarily given to describe the embodiments of the present disclosure, and should not be construed as being limited to the embodiments set forth herein, and it will be understood that the embodiments of the present disclosure are provided only to completely disclose the disclosure and cover modifications, equivalents or alternatives which come within the scope and technical range of the disclosure.

In the following description of the embodiments, terms, such as "first" and "second", are used only to describe various elements, and these elements should not be construed as being limited by these terms. These terms are used only to distinguish one element from other elements.

When an element or layer is referred to as being "connected to" or "coupled to" another element or layer, it may be directly connected or coupled to the other element or layer, or intervening elements or layers may be present. In contrast, when an element or layer is referred to as being "directly connected to" or "directly coupled to" another element or layer, there may be no intervening elements or layers present.

The implementation environment of a high-intensity focused ultrasound probe 100 according to the present disclosure will be described with reference to FIG. 1.

Referring to FIG. 1, a high-intensity focused ultrasound apparatus 200 according to one embodiment of the present disclosure may control the high-intensity focused ultrasound probe 100 to focus ultrasonic waves generated from the high-intensity focused ultrasound probe 100 to a specific point inside the body so as to transfer acoustic energy thereto. The high-intensity focused ultrasound apparatus 200 may change a focusing point by electrically controlling the transmission time of array-type transducer elements included in a transducer module of the high-intensity focused ultrasonic probe 100. The high-intensity focused ultrasound apparatus 200 may transfer high-intensity focused ultrasonic energy to the inside of the body for non-invasive face lifting procedures, skin tightening procedures, non-invasive subcutaneous fat layer reduction or removal procedures, and the like.

The high-intensity focused ultrasound apparatus 200 may include a controller including a processor configured to calculate the ultrasonic wave transmission time delay of the transducer elements of the high-intensity focused ultrasound probe 100 in order to change the focusing point.

In one embodiment, the high-intensity focused ultrasound apparatus 200 may include an input interface or an output interface for user input.

The user interface includes a user interface (UI) including a touch interface to receive information from a microphone and a user, the user interface may include not only a mouse and a keyboard but also mechanical and electronic interfaces implemented in the apparatus, and the method and form of the user interface are not particularly limited as long as a user command can be input therethrough. The electronic interface may include a display which enables touch input.

The output interface serves to deliver information to the user by displaying the output of the high-intensity focused ultrasound apparatus 200, and may include a display, LEDs, a speaker, and the like to display visual output, auditory output, or tactile output. The display may display the ultrasonic image of inner tissues of the body, if the high-intensity focused ultrasound probe 100 includes an imaging transducer module.

The high-intensity focused ultrasound apparatus 200 may include a peripheral interface for data transmission to various types of external devices connected thereto, and may include a memory card port, external device input/output (I/O) ports, and the like.

The high-intensity focused ultrasound apparatus 200 may be connected to the high-intensity focused ultrasound probe 100 by a cable or wirelessly to control the high-intensity focused ultrasound probe 100.

In one embodiment, the high-intensity focused ultrasound probe 100 may include a handpiece (or called a wand) 100b and a cartridge 100a. In this case, the ultrasound transducer module may be implemented within the cartridge 100a. In another embodiment, the high-intensity focused ultrasound probe 100 may be implemented by integrating the handpiece 100b and the cartridge 100a.

If the handpiece 100b and the cartridge 100a are implemented separately, an electrical connection terminal and a guide for physical coupling may be provided on the handpiece 100b so that the cartridge 100a may be coupled to the handpiece 100b. In one embodiment, the guide may be implemented in the shape of a bar or a protrusion protruding from the front end of the handpiece 100b in a direction in which the cartridge 100a is coupled thereto.

Referring to FIG. 2, the configuration of the high-intensity focused ultrasound probe 100 according to one embodiment of the present disclosure will be described.

The high-intensity focused ultrasound probe 100 may include a transducer module 110 which receives an electrical signal and generates ultrasonic waves, and a circuit 120 which inputs the electrical signal to the transducer module 110.

In one embodiment, the high-intensity focused ultrasound probe 100 may include a stepper motor which may one-dimensionally move the transducer module 110 in forward and rearward directions, i.e., the axial direction of the handpiece 100b, to focus ultrasonic waves on a plurality of focusing points (i.e., so that a plurality of ultrasonic focusing points may be arranged at regular intervals along the same line), if an array including a small number of transducer elements of the transducer module 110 is provided. In one embodiment, the high-intensity focused ultrasound probe 100 may include a sensor 130 which may determine the position of the transducer module 110. The sensor 130 may be an optical sensor, a roller ball sensor, or the like. In another embodiment, an encoder may be connected to a motor, which moves the transducer module 110, without using the sensor 130, and may calculate the moving displacement of the transducer module 110 and determine the current position of the transducer module 110. As the sensor 130, sensors for various purposes, which not only detect the movement of the transducer module 110 but also detect coupling between the handpiece 100b and the cartridge 100a (in this case, a pressure sensor or a switch configured to detect electrical connection), the angle of the handpiece 100b with respect to the ground, user's grip of the handpiece 100b, and the like, may be used in various ways.

In another embodiment, if an array including a large number of transducer elements is provided, ultrasonic waves may be focused on a plurality of focusing points in the forward and rearward directions, i.e., the axial direction of the handpiece 100b, by sequentially controlling some of the plurality of transducer elements and varying the transmission time of the controlled transducer elements. In this case, movement of the transducer module 110 may not be necessary.

In another embodiment, if an array including a large number of transducer elements is provided, the plurality of transducer elements (the transducer module) may be rotated at a predetermined angle around the longitudinal axis of the handpiece using the motor.

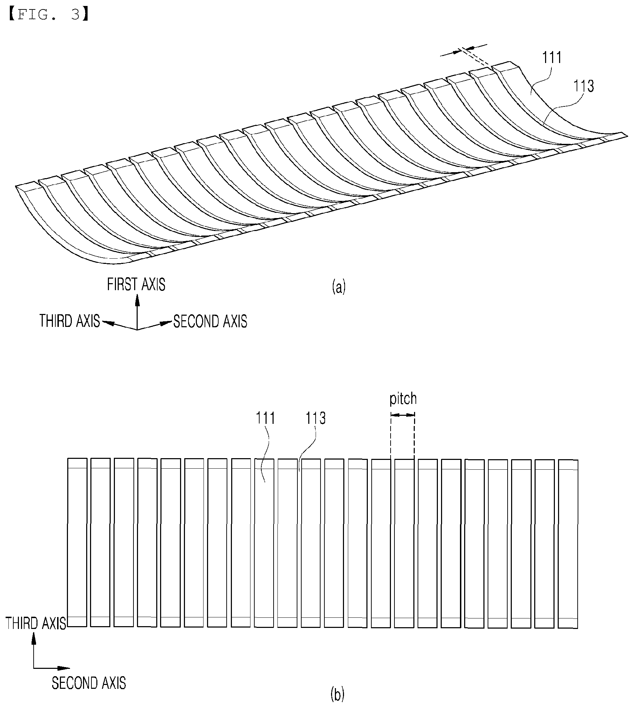

In another embodiment, in a one-dimensional array of the transducer elements, an axis in a direction in which the transducer elements are coupled may be perpendicular to the longitudinal axis of the handpiece 100b. Therefore, in this case, the transducer module may be moved so that the plurality of transducer elements arranged in the array moves in the longitudinal direction of the handpiece 100b (i.e., moves in a third axis direction in FIG. 3). In this case, ultrasonic energy is transferred simultaneously to a wider area, and may thus shorten a treatment time.

Referring to FIG. 3, the array-type high-intensity focused ultrasound transducer module according to one embodiment of the present disclosure will be described.

In this specification, an axis in a direction in which ultrasonic waves are generated from the plurality of transducer elements and are directed to a focusing point is referred to as a first axis (i.e., an axis in the height direction or the axial direction), an axis in a direction in which the plurality of transducer elements is arranged is referred to as a second axis (i.e., an axis in the lateral direction), and an axis in the longitudinal direction in which the respective transducer elements extend, that is, an axis perpendicular to the first and second axes, is referred to as a third axis (i.e., an axis in the elevation direction) . The first, second, and third axes are orthogonal to each other.

The array-type high-intensity focused ultrasound transducer module according to one embodiment of the present disclosure may be configured such that a plurality of transducer elements 111 is arranged in a one-dimensional array in the second axis direction with kerfs 113 interposed therebetween. As will be described later, the kerf may refer to spacing between adjacent transducer elements, and the sum of the width of the kerf and the width of the transducer element 111 in the second axis direction may be referred to as an element width (pitch). The level of grating lobes and the intensity of energy transmitted to the inside of the body may be varied depending on the pitch and the width of the kerf. Therefore, in the array-type high-intensity focused ultrasound transducers, the optimal pitch must be selected in advance to increase a main lobe level and to to minimize a grating lobe level.

Each transducer element 111 may have a geometrical shape which is concave in the first axis direction, and this geometrical structure is related to setting of the focusing point of ultrasonic waves generated from the transducer elements 111. Ultrasonic waves generated from each transducer element 111 by receiving an electrical signal may travel in the direction of forming the concave shape and be focused on a specific depth point inside the body to transfer energy.

Each transducer element 111 may extent lengthwise in the third axis direction. Therefore, the length of each transducer element 111 in the third axis direction may be longer than the width of each transducer element 111 in the second axis direction.

Ultrasonic waves generated from each transducer element 111 by receiving the electrical signal proceed in the direction of forming the concave shape of the transducer element 111 and are focused on a specific depth point inside the body, and energy transferred thereto (i.e., ultrasonic output energy of the transducer module) may be 0.5 J/cm² or more at the focusing point. Therefore, energy is transferred such that the temperature of the focusing point inside the body is about 60 or more degrees Celsius, thereby causing thermal variation at the focusing point inside the body.

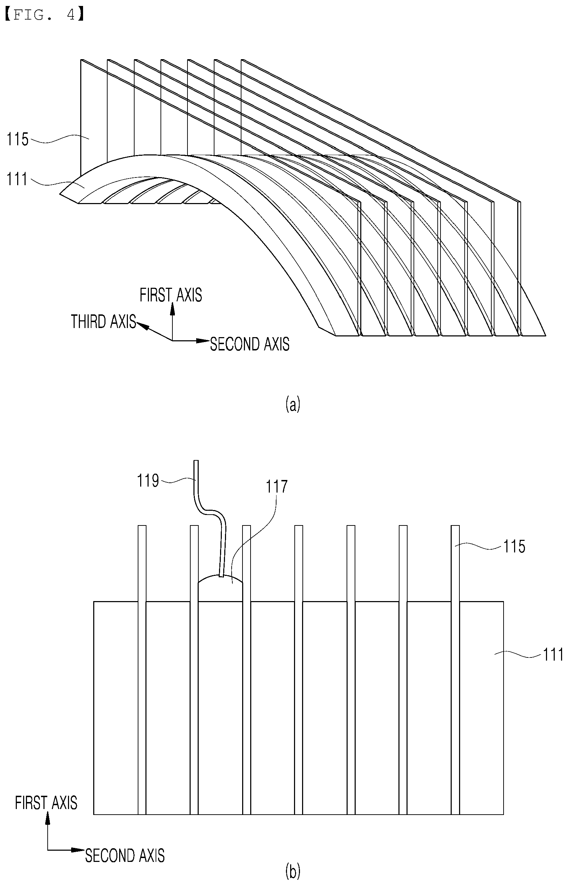

The array-type high-intensity focused ultrasound transducer module according to one embodiment of the present disclosure will be described with reference to FIG. 4.

The array-type high-intensity focused ultrasound transducer module according to one embodiment of the present disclosure may include separation members 115 disposed in the kerfs 113 between the high-intensity focused ultrasound transducer elements 111.

In one embodiment, the high-intensity focused ultrasound transducer elements 111 may not be directly connected to each other to be arranged in an array, but may be coupled in a fixed manner through the separation members 115.

In one embodiment, the separation members 115 may be formed of a material having thermal stability up to 300 or more degrees Celsius, and may be, for example, polyimide (PI). Therefore, the separation members 115 are formed of a material with little change in physical properties even when exposed to high temperatures for a long time to prevent the separation members 115 from being thermally deformed when a signal line is connected to the transducer elements 111 by soldering.

The signal line which applies an electrical signal may be connected to the rear surface of each of the plurality of transducer elements of the array-type high-intensity focused ultrasound transducer module according to one embodiment of the present disclosure, which is in the direction opposite to the ultrasonic wave focusing direction. Because the high-intensity focused ultrasound transducer elements apply a signal of high energy enough to cause thermal variation in a desired lesion, the high-intensity focused ultrasound transducer elements may instantly generate high-temperature heat, and in order to connect the signal line to the transducer elements, soldering performed at a melting point of about 300 or more degrees Celsius may be used.

In one embodiment, the length of the separation members 115 in the first axis direction may be longer than the length (thickness) of the transducers 111 in the first axis direction. Therefore, the separation members 115 may serve as dams which prevent molten lead from being connected to other elements when the signal line is connected to each of the transducer elements 111 by soldering.

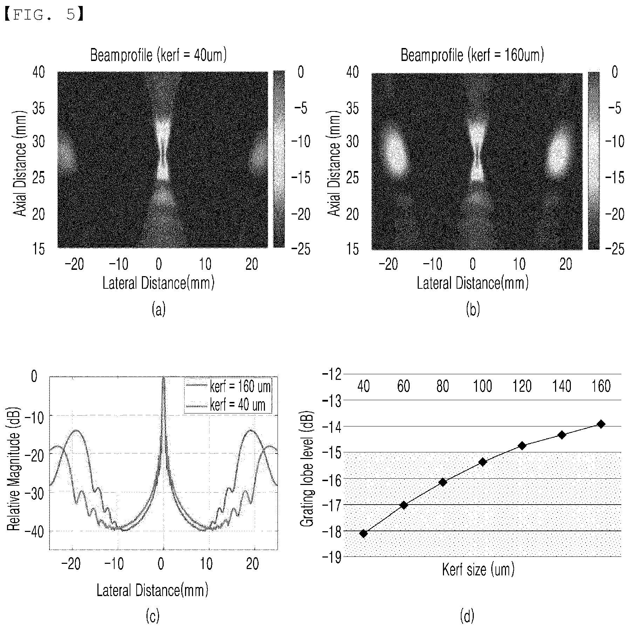

The kerf width (size) and the transducer element width of the array-type high-intensity focused ultrasound module according to one embodiment of the present disclosure will be described with reference to FIG. 5.

FIG. 5 shows results of a simulation on a grating lobe level depending on a kerf width. This simulation was conducted by changing the kerf width at 20 um intervals from 20 um to 160 um while fixing the transducer element width to about 460 um. Here, the range of the kerf width was set so that the level of the grating lobes is less than about -15 dB compared to the maximum energy. FIG. 5(a) shows a beam profile when the kerf width is 40 µm, and FIG. 5(b) shows a beam profile when the kerf width is 160 um. FIG. 5(c) shows line plots when the kerf width is 40 um and 160 um, respectively. FIG. 5(d) is a graph representing the relative level of the grating lobes depending on changes in the kerf width. It may be confirmed that, when the kerf width is 100 um, the relative level of the grating lobes is about -15.34 dB, and when the kerf width is 120 um, the relative level of the grating lobes is -14.74 dB, which is higher than -15 dB. Therefore, it may be seen that the kerf width may be increased to about 100 um under the corresponding conditions.

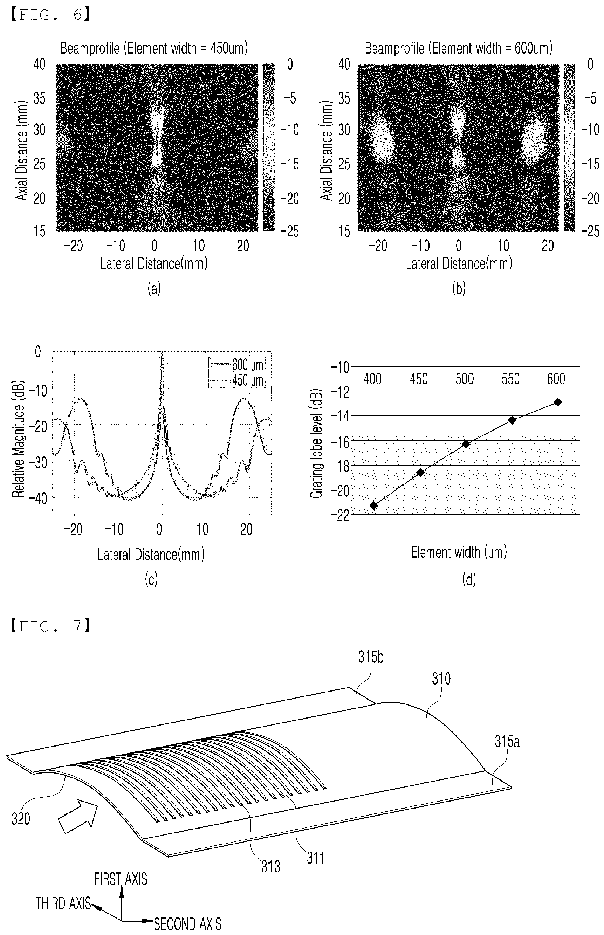

In FIG. 6, contrary to FIG. 5, a simulation was conducted by changing the element width at 50 um intervals from 400 um to 600 um while fixing the kerf width to 40 um. Through this, the range of the element width was set so that the level of the grating lobes is less than about -15 dB. FIG. 6(a) shows a beam profile when the element width is 450 µm, and FIG. 6(b) shows a beam profile when the element width is 600 um. FIG. 6(c) shows line plots when the element width is 450 um and 600 um, respectively. FIG. 6(d) is a graph representing the relative level of the grating lobes depending on changes in the element width. It may be confirmed that, when the element width is 500 um, the relative level of the grating lobes is about -16.26 dB, and when the element width is 550 um, the relative level of the grating lobes is -14.35 dB, which is higher than -15 dB, and therefore, it may be seen that the element width may be increased to 500-550 um under the corresponding conditions.

The results of the simulations of FIGs. 5 and 6 are the results of simulations conducted when the element width and the kerf width were fixed and a single frequency (4 MHz) was used, and the set ranges may vary depending on changes in various conditions. Because, through the simulations of FIGs. 5 and 6, it was confirmed that, as the element width and the kerf width increase, the level of the grating lobes may be increased and thus total energy may be decreased, the element width, the kerf width, and the pitch may be determined so that the total energy may be maximized.

An array-type high-intensity focused ultrasound probe according to another embodiment of the present disclosure will be described with reference to FIG. 7.

A detailed description of parts, which overlap those described previously, will be omitted, and the following description will focus on a transducer module.

A high-intensity focused ultrasound probe 100 may include a transducer module, and the transducer module may be configured such that transducer elements 311, which generate ultrasonic waves, may be formed at a plurality of regions of a curved transducer part 310 and 320 by kerfs 313.

The transducer module may include the curved transducer part 310 and 320, and bent parts 315a and 315b which are bent in the direction opposite to the ultrasonic wave focusing direction from at least one of both ends of the transducer part 310 and 320 in the third axis direction. The curved transducer part 310 and 320 may have a concave shape in the first axis direction, which is the focusing direction of ultrasonic waves.

In one embodiment, an electrical signal may be applied to the transducer elements 311, formed at a plurality of regions by separating portions of the curved transducer part 310 and 320 due to the kerfs 313, to generate ultrasonic waves, and a signal line configured to apply the electrical signal therethrough may be connected to each of the transducer elements 311 on the rear surface 310 of the curved transducer part in the direction opposite to the ultrasonic wave focusing direction, by soldering. In one embodiment, separation members, as shown in FIG. 4, may be disposed in the kerfs 313.

In one embodiment, a ground electrode may be formed on at least a portion of the front surface 320 of the curved transducer part in the ultrasonic wave focusing direction, and may be formed using a sputtering method to reduce ultrasonic attenuation. The ground electrode may be formed on at least a portion of the front surface 320 in the ultrasonic wave focusing direction from the transducer elements 311 formed at the plurality of regions.

The plurality of transducer elements 311 formed at the plurality of regions by separating the portions of the curved transducer part 310 and 320 indicates portions separated from each other by the kerfs 310 extending lengthwise in the third axis direction, and therefore, at least one end of both ends of the transducer elements 311 in the third axis direction may be connected to one another.

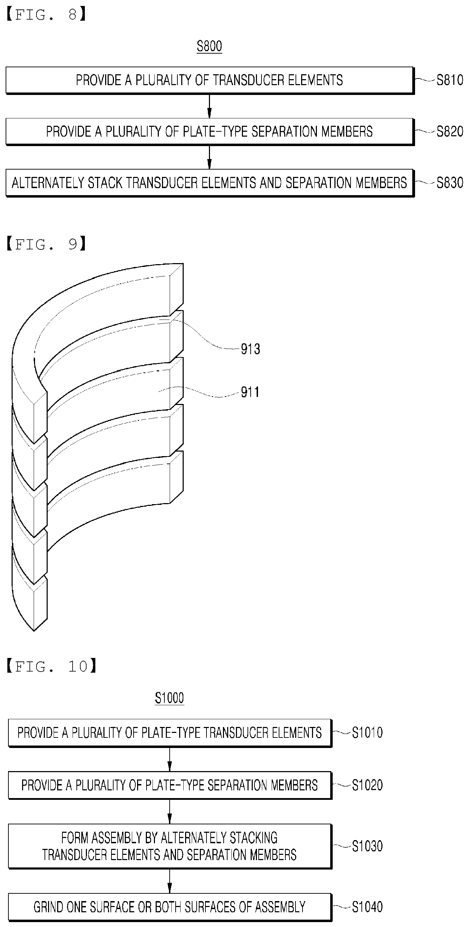

Methods of manufacturing array-type high-intensity focused ultrasound probes according to various embodiments of the present disclosure will be described with reference to FIGs. 8 to 13.

First, referring to FIGs. 8 and 9, a method of manufacturing a high-intensity focused ultrasound probe using a stacking method will be described.

A plurality of transducer elements 911, which has a predetermined thickness in the first axis direction, in which, when an electrical signal is applied, ultrasonic waves are generated and focused, and a predetermined curvature to be concave in the first axis direction, and extends lengthwise in the third axis direction perpendicular to the first axis direction, are provided (S810).

A plurality of plate-type separation members having a predetermined second thickness is provided (S820), and in this case, the plate-type separation members may be formed of a material having thermal stability up to 300 or more degrees Celsius, and may be, for example, polyimide.

A transducer module is manufactured by alternately stacking the plurality of curved transducer elements and the plurality of plate-type separation members in the second axis direction orthogonal to the first axis direction and the third axis direction so that the plate-type separation members are disposed in kerfs 911 (S830), and thereafter, a signal line may be connected to each of the rear surfaces (i.e., the surfaces in a direction opposite to the ultrasonic wave focusing direction) of the curved transducer elements, and a ground electrode may be formed on the front surfaces of the curved transducer elements. The stacking method has the effect of excellent ease of manufacture.

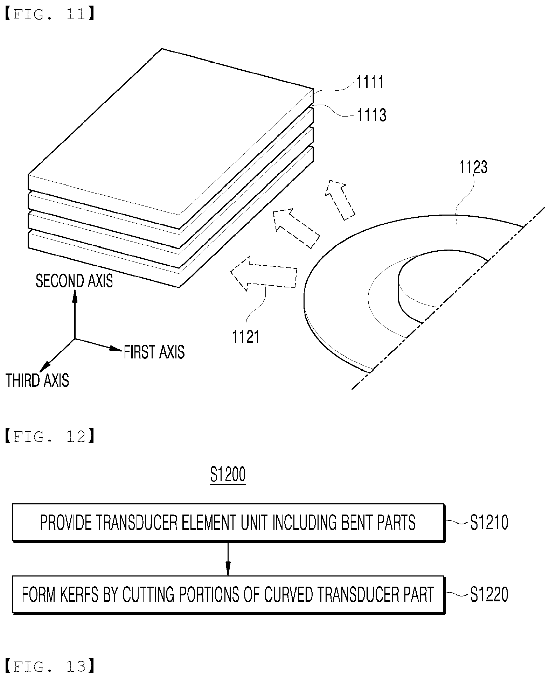

Referring to FIGs. 10 and 11, a method of manufacturing a high-intensity focused ultrasound probe using a polishing (or grinding) method will be described.

A plurality of plate-type transducer elements 1111 having a predetermined thickness in the second axis direction is provided (S1010), and a plurality of plate-type separation members 1113 having another predetermined thickness is provided (S1020). Thereafter, an assembly is formed by alternately stacking the plurality of transducer elements 111 and the plurality of separation members 1113 in the second direction (S1030), and one surface or both surfaces of the assembly perpendicular to the first axis are ground 1121 using a grinding machine 1121 (S1040). Here, the curvature of a grinding blade of the grinding machine 1123 is set to form a geometrical structure considering an ultrasonic wave focusing point. One surface of the assembly may be ground to have a predetermined thickness in the first axis direction, which is the direction in which ultrasonic waves are generated and focused, and a predetermined curvature to be concave in the first axis direction, and the other surface (i.e., the rear surface) of the assembly in the opposite direction (the surface of the assembly in the direction opposite to the ultrasonic wave focusing direction) may be ground to have a predetermined curvature to be convex in the first axis direction. The grinding method has the effect of excellent ease of manufacture and low sensitivity.

Referring to FIGs. 12 and 13, a method of manufacturing a high-intensity focused ultrasound probe using a cutting method will be described.

A transducer element unit 1300 including a curved transducer part 1331 and 1333 configured such that at least a portion thereof has a concave shape in the first axis direction, which is the focusing direction of ultrasonic waves, and bent parts 1310a and 1310b which are bent in the direction opposite to the ultrasonic wave focusing direction from both ends of the transducer part 1331 and 1333 in the third axis direction perpendicular to the first axis direction is provided (S1210).

A plurality of kerfs 1313, which extends lengthwise in the third axis direction and is arranged in the second axis direction perpendicular to the first axis direction and the third axis direction, is formed in the curved transducer part 1331 and 1333 by cutting at least portions of the curved transducer part 1331 and 133 through a cutting machine 1340 (S122).

An electrical signal may be applied to a plurality of transducer elements 1311 formed at a plurality of regions by separating the portions of the curved transducer part 1311 and 1333 due to the kerfs 1313, to generate ultrasonic waves, and a signal line configured to apply the electrical signal therethrough may be connected to the rear surface 1333 in the opposite to the ultrasonic wave focusing direction, by soldering. In one embodiment, separation members, as shown in FIG. 4 may be disposed in the kerfs 1313. In one embodiment, a ground electrode may be formed on the front surface 1331 of the curved transducer part 1331 and 1333 using the sputtering method.

In the specification (particularly, in the claims) of the present disclosure, use of the term "above" and similar referential terms may refer to both the singular and the plural. In addition, when a range is stated in the present disclosure, the statement includes the invention to which individual values within the range are applied (unless there is a statement to the contrary), and is the same as a statement of the individual values constituting the range in the detailed description of the invention.

Unless there is a statement of an explicit order or a statement to the contrary regarding steps constituting the method according to the present disclosure, the steps may be performed in any appropriate order. The present disclosure is not necessarily limited by the described order of the steps. Use of any examples or illustrative terms (for example, etc.) in the present disclosure is merely to describe the present disclosure in detail, and unless limited by the claims, the scope of the present disclosure is not limited by the examples or illustrative terms. Further, those skilled in the art will appreciate that various modifications, combinations, and changes may be made according to design conditions and factors within the scope of the appended claims or their equivalents.

Therefore, the spirit of the present disclosure should not be limited to the above-described embodiments, and the scope of the appended claims described below as well as all scopes equivalent to or equivalently changed from the claims are within the scope of the spirit of the present disclosure.

## Claims

1. A method of manufacturing a high-intensity focused ultrasound (HIFU) probe, comprising:
manufacturing a transducer module configured such that a plurality of transducer elements having a concave shape in a first axis direction, which is a focusing direction of ultrasonic waves generated from the transducer module by receiving an electrical signal, is arranged in a second axis direction perpendicular to the first axis direction with kerfs interposed between the transducer elements; and
disposing a circuit configured to input the electrical signal to the transducer module,
wherein each of the plurality of transducer elements is formed in a geometrical structure configured to cause the ultrasonic waves generated by receiving the electrical signal to be focused on a predetermined point, and has a shape configured such that a length thereof in a third axis direction perpendicular to the first axis direction and the second axis direction is longer than a length thereof in the second axis direction.

2. The method according to claim 1, wherein ultrasonic output energy of the transducer module is 0.5 J/cm² or more at the focusing point.

3. The method according to claim 1, further comprising:
disposing separation members in the kerfs,
wherein a length of the separation members in the first axis direction is longer than a length of the transducer elements in the first axis direction.

4. The method according to claim 3, further comprising:
connecting a signal line configured to apply the electrical signal to a rear surface of each of the plurality of transducer elements in a direction opposite to the focusing direction of the ultrasonic waves, by soldering.

5. The method according to claim 1, further comprising:
disposing separation members formed of a material having thermal stability up to 300 or more degrees Celsius in the kerfs.

6. The method according to claim 5, wherein the separation members are formed of polyimide.

7. The method according to claim 1, wherein manufacturing the transducer module comprises:
forming the plurality of transducer elements so that the plurality of transducer elements is not directly connected to one another in the second axis direction.

8. The method according to claim 1, wherein manufacturing the transducer module comprises:
forming the plurality of transducer elements so that at least one end of both ends of the plurality of transducer elements in the third axis direction is connected to one another.

9. The method according to claim 8, further comprising:
forming a ground electrode on at least a portion of a front surface of each of the plurality of transducer elements in the focusing direction of the ultrasound waves.

10. The method according to claim 9, further comprising:
forming a bent part by bending an extension from at least one end of both ends of each of the plurality of transducer elements in the third direction, in a direction opposite to the focusing direction of the ultrasonic waves.

11. A method of manufacturing a high-intensity focused ultrasound (HIFU) probe, comprising:
forming a transducer module configured to receive an electrical signal and generate ultrasonic waves; and
disposing a circuit configured to input the electrical signal to the transducer module,
wherein forming the transducer module comprises:
forming a curved transducer part having a concave shape in a first axis direction, which is a focusing direction of the generated ultrasonic waves;
forming a plurality of kerfs, arranged in a second axis direction perpendicular to the first axis direction in the curved transducer part, provided to extend lengthwise in a third axis direction perpendicular to the first axis direction and the second axis direction, and configured to separate at least portions of the curved transducer part so as to form transducer elements at a plurality of regions; and
forming a bent part, configured to be bent in a direction opposite to the focusing direction of the ultrasonic waves, at least one end of both ends of the transducer part in the third axis direction.

12. The method according to claim 11, further comprising:
forming a ground electrode on at least a portion of a front surface of the curved transducer part in the focusing direction of the ultrasound waves;
connecting a signal line configured to apply the electrical signal to at least a portion of a rear surface of the curved transducer part opposite to the front surface thereof by soldering; and
disposing separation members having a length in the first axis direction longer than a length of the curved transducer part in the first axis direction, in the kerfs.

13. A method of manufacturing a high-intensity focused ultrasound (HIFU) probe, comprising:
providing a plurality of transducer elements having a predetermined first thickness in a first axis direction and a predetermined curvature to be concave in the first axis direction, and configured to extend lengthwise in a third axis direction perpendicular to the first axis direction;
providing a plurality of plate-type separation members having a predetermined second thickness; and
alternately stacking the plurality of transducer elements and the plurality of separation members in a second direction perpendicular to the first axis direction and the third axis direction.

14. A method of manufacturing a high-intensity focused ultrasound (HIFU) probe, comprising:
providing a plurality of plate-type transducer elements having a predetermined first thickness in a second axis direction;
providing a plurality of plate-type separation members having a predetermined second thickness;
forming an assembly by alternately stacking the plurality of plate-type transducer elements and the plurality of plate-type separation members in the second axis direction; and
grinding one surface or both surfaces of the assembly perpendicular a first axis direction so that the plurality of plate-type transducer elements has a predetermined curvature to be concave in the first axis direction perpendicular to the second axis direction and extends in a third axis direction perpendicular to the first axis direction and the second axis direction.

15. A method of manufacturing a high-intensity focused ultrasound (HIFU) probe, comprising:
providing a transducer element unit comprising a curved transducer part configured such that at least a portion thereof has a concave shape in a first axis direction, which is a focusing direction of ultrasonic waves, and bent parts configured to bent in a direction opposite to the focusing direction of the ultrasonic waves from both ends of the transducer part in a third axis direction perpendicular to the first axis direction; and
forming a plurality of kerfs, configured to extend lengthwise in the third axis direction and arranged in a second axis direction perpendicular to the first axis direction and the third axis direction, in the curved transducer part by cutting at least portions of the curved transducer part.
